# EUROPEAN PATENT APPLICATION

(11) **EP 1 932 517 A2**
(43) Date of publication of application: **18.06.2008**
(21) Application number: 06077189.6
(22) Date of filing: 11.12.2006
(51) Int. Cl.: A61K 9/127, A61K 31/216

(54) **Liposomes containing a polyphenol derivative such as caffeic acid and a method of post-loading thereof**

(71) Applicant: Universiteit Utrecht Holding B.V., 3584 CM Utrecht (NL)
(72) Inventor: The designation of the inventor has not yet been filed
(74) Representative: van Loon, C.J.J.

(57) **Abstract**

The present invention relates to liposomal compositions comprising polyphenol, and especially to the use of such compositions in the treatment of cancer or in the inhibition of cancer growth and in the treatment of inflammatory and autoimmune conditions. More specifically, the invention relates to a method for targeting a polyphenol to tumor tissue, or to sites in the body requiring anti-inflammatory activity or activity against autoimmune conditions. Furthermore the present invention describes a method of post-loading a polyphenol derivative such as caffeic acid into a liposome.

## Description

The present invention relates to compositions comprising polyphenol, and especially to the use of such compositions in the treatment of cancer or in the inhibition of cancer growth and in the treatment of inflammatory and autoimmune conditions. More specifically, the invention relates to a method for targeting a polyphenol to tumor tissue, or to sites in the body requiring anti-inflammatory activity or activity against autoimmune conditions. Even more specifically, the present invention aims to provide the use of such compositions in the treatment of non-lymphatic cancers, and preferably solid (non-hematological) malignancies and metastases (solid primary and secondary tumors), and is hence in the field of oncology. In the treatment of non-solid tumors or lymphatic cancer types, e.g. chronic and acute lymphatic leukaemia, Hodgkin and non-Hodgkin lymphomas, polyphenols can however also be used.

Polyphenols form a large family of natural compounds widely distributed in plant foods. Polyphenols are responsible for the coloring of many plants, leafs and fruits. High levels of polyphenols can generally be found in fruit skins; for example high percentages are found in grape skin, apple skin and orange skin. The last decade, especially green tea polyphenols and polyphenols from wine have attracted attention.

Research indicates that there is a range of different polyphenols that have beneficial effects at the level of inflammation and cancer. The oncoprotective and anti-inflammatory / immunomodulatory properties of exogenous antioxidants have been documented in a number of epidemiological, intervention and in vitro studies. However, the mechanisms implicated are far from being clarified. Antioxidant effects, steroid receptor binding, direct interaction with intracellular elements and signaling systems (antiproliferative and apoptotic effects) have all been proposed as possible mechanisms for the mediation of the oncoprotective and anti-inflammatory / immunosuppressive effect of these agents.

There are considerable literature data concerned with both the bioavailability of dietary polyphenols and with their efficacy as health promoting compounds. Dietary polyphenols are poorly bioavailable and are extensively metabolised. Only a small portion of the ingested dose reaches the systemic circulation and is excreted in urine. Bioavailabilities are highly variable depending on structure and conjugation (e.g. to sugars): less than 0.1% for most anthocyanins (coloured flavonoids in berries, red wine), 1-5% for quercetin (red wine, apples, onions) and 10-30% for flavanones (citrus) and flavanols (red wine, tea, cocoa). Polyphenols are metabolised during absorption and typically the forms present in plasma differ from those ingested. Metabolism occurs predominantly in the intestine (gut epithelial cells and colonic microflora) and the liver. The maximum concentration in plasma rarely exceeds 1 microM after the consumption of 10-100 mg of a single phenolic compound. The products of human metabolism are glucuronides and sulfates of the polyphenol aglycone and methylated derivatives. Products derived from the colonic microflora are simple phenolic acids.

Because polyphenol bioavailability after oral administration is poor, target tissue concentrations of polyphenols that are effective in and against inflammatory and/or tumor cells cannot be achieved by the oral route. Intravenous administration of free polyphenols will most likely be only poorly effective as most polyphenols appear to be rapidly metabolized in circulation.

Hence, there is a need in the art for a more effective therapeutic based on polyphenols for inflammatory conditions and cancer. More specifically, there remains a need for a pharmaceutical composition that is more effective in delivering polyphenols to target tissues such as sites of inflammation and tumors.

In addition, the amounts of polyphenol required to obtain any effect, if at all, are high. That is, if one were to use polyphenols in cancer- or tumor-therapy, one would need high dosing (by way of typical example: 1.5-2 g per day for resveratol). for prolonged periods of time to obtain significant tumor growth inhibition. This would intensify all kinds of side-effects and will most likely cause toxicities to arise.

The present invention focuses on the aspect of providing measures to predictably exploit the full potential of polyphenols. It is, hence, an objective of the present invention to find a method to target polyphenols to tumor tissue for or in the treatment, retardation or inhibition of cancer; or to find a method to target the polyphenols to sites requiring anti-inflammatory activity and activity against autoimmune conditions.

In accordance with the present invention, it has been found that by selectively delivering polyphenols to tumor tissue so that local drug concentrations are increased, a pharmacological effect towards cell types involved in the progression of cancer is achieved. Corresponding effects are found by selectively delivering polyphenols to inflamed tissue or tissue affected by autoimmune diseases so that local drug concentrations are increased, and a pharmacological effect towards cell types involved in the progression of inflammation / autoimmune conditions is achieved.

It is therefore an object of the present invention to provide a pharmaceutical composition for parenteral administration, comprising polyphenol.

A further object of the present invention is to provide a use of a pharmaceutical composition, comprising polyphenol for the preparation of a medicament effective in the site-specific treatment of inflamed tissues or regions, or of cancer, after parenteral administration.

The present invention provides compositions and methods useful for the treatment of inflammatory disorders and cancer in which polyphenols or polyphenol derivatives are encapsulated in the interior of long-circulating liposome formulations, which can be administered parenterally to a patient.

More particularly, the present invention relates to a pharmaceutical composition for parenteral administration, the composition comprising colloidal carriers composed of long-circulating, non-charged vesicle-forming lipids, which colloidal carriers contain one or more polyphenol or polyphenol derivatives entrapped in the carrier interior.

In this pharmaceutical composition, the colloidal carriers preferably have a neutral or negative charge at physiological conditions, and are preferentially liposomes, nano-capsules or polymeric micelles.

The colloidal carriers suitably have a selected mean particle diameter in the size range between about 40 - 200 nm.

From a compositional point of view the colloidal carriers preferably include up to 20 mole percent of an amphipathic vesicle-forming lipid derivatized with a water-soluble polymer, optionally up to 10 mol% of negatively charged vesicle-forming lipids, and optionally up to 50 mole percent of a sterol.

In a second aspect, the present invention relates to the use of a pharmaceutical composition of the invention for the treatment, and especially for the site-specific treatment of inflammatory or autoimmune disorders and cancer.

In a third aspect, the present invention relates to the use of a pharmaceutical composition according to the invention for the preparation of a medicament effective in the treatment, and especially for the site-specific treatment of inflammatory or autoimmune disorders and cancer. Preferably, this use is for the site-specific treatment of inflamed tissues or regions after parenteral administration, wherein the polyphenol is used in the medicament in a water soluble form.

Suitably, rheumatoid arthritis and multiple sclerosis may be treated according to the present invention.

In yet a further aspect, the invention is directed to a method for loading into long-circulating colloid carriers, a polyphenol or polyphenol derivative having a deprotonatable hydroxyl group, said method comprising: (a) preparing a suspension of long-circulating colloid carriers, preferably liposomes, having a greater concentration of metal cations inside the liposomes than outside the liposomes, said metal cations being capable of forming complexes or precipitates at relatively high pH, that is a pH of 7 and higher, with the negatively charged polyphenol or polyphenol derivatives, (b) adding negatively charged polyphenol or polyphenol derivatives compound to the said suspension, and (c) achieving uptake of the compound into the liposomes, to a final concentration of compound within the liposomes which is greater than that outside the liposomes.

The long-circulation liposomes according to the present invention have a circulation half life of at least 3 hours, and especially at least 6 hours, the circulation half life being defined as the time at which the second linear phase of the logarithmic liposomal clearance profile reaches 50% of its initial concentration, which is the extrapolated plasma concentration at t=0.

Long-circulating liposomes in this invention are composed of non-charged vesicle-forming lipids, optionally including up to 20 mole percent of an amphipathic vesicle-forming lipid derivatized with a water-soluble polymer and optionally including not more than 10 mole percent of negatively charged vesicle-forming lipids, the liposomes having a selected mean particle diameter in the size range between about 40-200 nm.

These compositions lead to an increased localization and improved retention of the encapsulated polyphenol or polyphenol derivatives at diseased target tissues after a single parenteral injection. and as a consequence thereof, increased beneficial effects are observed as compared to the unencapsulated compound.

The methods of the present invention are useful for the treatment or prevention of inflammatory and autoimmune disorders, and cancer, in all mammalian subjects, including particularly human patients.

More in detail, the invention provides compositions and methods useful for treatment or prevention of a range of inflammatory and autoimmune disorders, which include but are not limited to rheumatoid arthritis, osteoarthritis and related joint inflammatory conditions, inflammatory bowel disorders including but not limited to Crohn's disease and colitis ulcerosa, multiple sclerosis and other neurologic inflammatory conditions, tissue transplant rejection, psoriasis and other skin inflammatory conditions, asthma, and rarer forms of inflammatory and autoimmune disorders including but not limited to aplastic anemia, autoimmune hepatitis, goodpasture's syndrome and lupus erythematosus.

The present invention can also be used to treat cancers of any of a wide variety of types, including both solid tumors and non-solid tumors such as leukemia and lymphoma. Carcinomas, sarcomas, myelomas, lymphomas, and leukemias can all be treated using the present invention, including those cancers which have a mixed type. Specific types of cancer that can also be treated include, but are not limited to: adenocarcinoma of the breast or prostate; all forms of bronchogenic carcinoma of the lung; myeloid; melanoma; glioma, hepatoma; neuroblastoma; papilloma; apudoma; choristoma; branchioma; sarcoma; neoplasms (e. g. , bone, breast, digestive system,colorectal, liver, pancreatic, pituitary, testicular, orbital, head and neck, central nervous system, acoustic, pelvic, respiratory tract, and urogenital) ; neurofibromatosis, and cervical dysplasia), and the like.

Polyphenols that can be used in the compositions of the present invention can be classified into the following categories:

### 1. Phenolic acids:

Phenolic acids form a diverse group that includes the widely distributed hydroxybenzoic and hydroxycinnamic acids, as well as other simple molecules such as caffeic acid, vanillin, and coumaric acid.

Phenolic acids are plant metabolites widely spread throughout the plant kingdom. Recent interest in phenolic acids stems from their potential protective role, through ingestion of fruits and vegetables, against oxidative damage diseases (coronary heart disease, stroke, and cancers).

Phenolic compounds are essential for the growth and reproduction of plants, and are produced as a response for defending injured plants against pathogens. Phenolic acid compounds seem to be universally distributed in plants. Phenolic acids may occur in food plants as esters or glycosides conjugated with other natural compounds such as flavonoids, alcohols, hydroxyl-fatty acids, sterols, and glucosides.

Coffee is particularly rich in bound phenolic acids, such as caffeic acid, ferulic acid, and p-coumaric acid. Phenolic acids found in blueberries include gallic acid, p-hydroxybenzoic acid, caffeic acid, p-coumaric acid and vanillic acid.

Phenolic acids have been the subject of a great number of chemical, biological, agricultural, and medical studies.

Examples from the diverse group of phenolic acids include
a. hydroxycinnamic acids: e.g., p-coumaric acid, caffeic acid, ferulic acid. Hydroxycinnamic acid compounds occur most frequently as simple esters with hydroxy carboxylic acids or glucose. They include coumaric acid, caffeic acid and ferulic acid. p-Coumaric acid can be found in a wide variety of edible plants such as peanuts, tomatoes, carrots, and garlic. It is a crystalline solid that is slightly soluble in water, but well soluble in ethanol and diethyl ether. p-Coumaric acid is an antioxidant and is believed to reduce the risk of stomach cancer by reducing the formation of carcinogenic nitrosamines. Caffeic acid is a carboxylic acid found in many fruits, vegetables, seasonings and beverages, principally in conjugated forms such as chlorogenic acid. This compound is a secondary plant metabolite produced in dandelion, yarrow, horsetail and whitethorn. The amount of caffeic acid is strongly dependent on the source from which the plant derives. Caffeic acid phenethyl ester (CAPE), an active component of propolis from honeybee hives, is known to have antimitogenic, anticarcinogenic, anti-inflamatory, and immunomodulatory properties. As an antioxidant, ferulic acid may neutralize free radicals such as reactive oxygen species (ROS). ROS may be involved in DNA damage and accelerated cell aging. Animal studies and *in vitro* studies suggest that ferulic acid may have direct antitumor activity against breast cancer and liver cancer. Ferulic acid may have pro-apoptotic effects in cancer cells, thereby leading to the destruction of these cancer cells. Ferulic acid may be effective at preventing cancer induced by exposure to the carcinogenic compounds benzopyrene and 4-nitroquinoline 1-oxide. It is however noted that there are no randomized controlled trials done with human participants, which means that the results of these studies may not be directly applicable to human use. If added to a topical preparation of ascorbic acid and vitamin E, ferulic acid may reduce oxidative stress and formation of thymidine dimers in skin.
b. hydroxybenzoid acids: e.g., p-hydroxybenzoic acid, gallic acid, ellagic acid. Hydroxybenzoic acid compounds are present mainly in the form of glucosides. They include 4-hydroxybenzoid acid, gallic acid and ellagic acid. The highest levels of ellagic acid are found in raspberries, strawberries, and pomegranates. Research in cell cultures and laboratory animals shows that ellagic acid may slow the growth of some tumors caused by certain carcinogens. While this is promising, at this time there is no reliable evidence from human studies showing that ellagic acid in any form can prevent or treat cancer. Further research is needed to determine what benefits it may have. Ellagic acid seems to have some anti-cancer properties. It can act as an antioxidant, and has been found to cause apoptosis (cell death) in cancer cells in the laboratory. There are also reports that it may help the liver to break down or remove some cancer-causing substances from the blood. Unfortunately, many substances showing promise against cancer in the laboratory and animal studies have not been found to be useful in humans.
c. rosmarinic acid. Rosmarinic acid is found in large quantities in oregano, lemon balm, sage, marjoram, and rosemary. Rosmarinic acid has antioxidant, anti-inflammatory and antimicrobial activities. The antioxidant activity of rosmarinic acid is stronger than that of vitamin E. Rosmarinic acid helps to prevent cell damage caused by free radicals, thereby reducing the risk for cancer and atherosclerosis. Perilla, rich in rosmarinic acid, is used for its anti-allergic activity. Unlike antihistamines, rosmarinic acid prevents the activation of immune responder cells, which cause swelling and fluid formation.

### 2. Flavonoids:

Flavonoids form a subclass of polyphenols. Flavonoids are widely distributed in nature, albeit not uniformly. The term "flavonoid" refers to a class of plant secondary metabolites based around a phenylbenzopyrone structure. Flavonoids are most commonly known for their antioxidant activity. Flavonoids are also commonly referred to as bioflavonoids - these terms are equivalent and interchangeable, since all flavonoids are biological in origin.

Flavonoids are widely distributed in plants fulfilling many functions including producing yellow or red/blue pigmentation in flowers and protection from attack by microbes and insects. The widespread distribution of flavonoids, their variety and their relatively low toxicity compared to other active plant compounds (for instance alkaloids) mean that many animals, including humans, ingest significant quantities in their diet.

Flavonoids have been found in high concentrations in butterflies and moths sequestered from dietary intake at the larval stage and then stored in adult tissues.

Flavonoids have been referred to as "nature's biological response modifiers" because of strong experimental evidence of their inherent ability to modify the body's reaction to allergens, viruses, and carcinogens. They show anti-allergic, anti-inflammatory, anti-microbial and anti-cancer activity.

In addition, flavonoids act as powerful antioxidants, protecting against oxidative and free radical damage.

Consumers and food manufacturers have become interested in flavonoids for their medicinal properties, especially their potential role in the prevention of cancers and cardiovascular disease. The beneficial effects of fruit, vegetables, tea and red wine have been attributed to flavonoid compounds rather than to known nutrients and vitamins.

The group of flavonoids is divided in the following subclasses:
a. flavones: e.g., luteolin, apigenin, flavoxate, diosmin. ad a. Flavones are 4-ketone derivatives of flavonoids and include apigenin, diosmin, flavoxate, luteolin. Diosmin is a pharmacologically very active compound. At the level of inflammatory and immune disorders diosmin reduces capillary hyperpermeability and increases capillary resistance by protecting the microcirculation from damaging processes. Diosmin also reduces the expression of endothelial adhesion molecules (ICAM, VCAM), and inhibits the adhesion, migration, and activation of leukocytes at the capillary level. This leads to a reduction in the release of inflammatory mediators, principally oxygen free radicals and prostaglandins. Luteolin is thought to play an important role as an antioxidant, a free radical scavenger, an agent in the prevention of inflammation, a promoter of carbohydrate metabolism, and an immune system modulator. These characteristics of luteolin are also believed to play an important part in the prevention of cancer. Multiple research experiments describe luteolin as a biochemical agent that can dramatically reduce inflammation and the symptoms of septic shock.
b. flavanones: e.g., hesperetin, naringenin. Flavanones are flavones hydrogenated at the 2 and 3-position (lacking the double bonds). They include hesperetin and naringenin. Hesperidin is a flavonoid glycoside found abundantly in citrus fruits. Its aglycone form is called hesperetin. Hesperidin is believed to play a role in plant defense. It acts as an antioxidant according to *in vitro* studies. In human nutrition it contributes to the integrity of the blood vessels. Various preliminary studies reveal novel pharmaceutical properties. Heperidin reduced cholesterol and blood pressure in rats. In a mouse study large doses of the glucoside hesperidin decreased bone density loss. Another animal study showed protective effects against sepsis. Hesperitin has anti-inflammatory effects. Naringenin is a flavanone that is considered to have a bioactive effect on human health as antioxidant, free radical scavenger, anti-inflammatory, carbohydrate metabolism promoter, and immunity system modulator. This substance has also been shown to repair DNA. Scientists exposed cells to 80 micomoles of naringenin per liter, for 24 hours, and found that the amount of hydroxyl damage to the DNA was reduced by 24% in that very short period of time. Unfortunately, this bioflavonoid is difficult to absorb on oral ingestion. In the best case scenario, only 15% of ingested naringenin will get absorbed in the human gastrointestinal tract.
c. flavonols: e.g., quercetin, myricetin, simylarin. Flavonols are 3-hydroxy 4-ketone-derivatives of flavonoids and include quercetin and myricetin. Quercetin is a flavonoid that forms the "backbone" for many other flavonoids, including the citrus flavonoids rutin, hesperidin, naringin and tangeritin. In studies, quercetin is found to be the most active of the flavonoids, and many medicinal plants owe much of their activity to their high quercetin content. Quercetin has demonstrated significant anti-inflammatory activity because of direct inhibition of several initial processes of inflammation. Quercetin also shows remarkable anti-tumour properties. Quercetin may have positive effects in combating or helping to prevent cancer, prostatitis, heart disease, cataracts, allergies/inflammations, and respiratory diseases such as bronchitis and asthma. Myricetin is a naturally-occurring flavonoid found in many grapes, berries, fruits, vegetables, herbs, as well as other plants. Walnuts are a rich dietary source. Myricetin has antioxidant properties. *In vitro* research suggests that myricetin in high concentrations can modify LDL cholesterol such that uptake by white blood cells is increased. Simylarin is found in milk thistle. Silmarin is a antioxidant or free radical scavenger. Skin care products often contain silymarin because it antioxidant activity may reduce the risk for skin cancer risk. Silibinin (silybin) is the major active constituent of silymarin. It is used in treatment and prevention of liver diseases because of its hepatoprotective (antihepatotoxic) properties. Clinical tests showed also its ability to protect against certain types of cancer (skin and prostate), probably due to its antioxidant properties. Silymarin provides protection against different stages of UVB-induced carcinogenesis. Silymarin protects the liver by promoting the growth of new liver cells. By inhibiting lipid peroxidation silymarin helps to reduce or prevent liver damage caused by alcohol, poisonous mushrooms, drugs and other toxins. Silymarin also helps with the digestion of fats. Silymarin has also anti-atherosclerotic activity, by inhibiting the expression of adhesion molecules.
d. flavan-3-ols: e.g., catechin and derivatives thereof, epicatechin and derivatives thereof, theaflavin. Catechins or flavan-3-ols are 3-hydroxy-derivatives of flavonoids (without the 4-ketone group) and include catechin, epicatechin and theaflavin. Catechins are powerful anti-oxidants. The best sources of catechins are white and green tea. Catechins are linked to evidence of fighting tumors as well as enhancing immune system function, due to their polyphenol antioxidant character, which is well established in scavenging reactive oxygen species. Tea contains four main catechin substances: catechin (EC), epicatechin gallate (ECg), epigallocatechin (EGC) and epigallocatechin gallate (EGCg). Catechin gallates are esters of a catechin and gallic acid whereas gallocatechins have the same carbon skeleton as other catechins but have an extra hydroxyl function. Epigallocatechin gallate (EGCG) is the one most abundant catechins in tea. EGCG as an antioxidant is about 25-100 times more potent than vitamins C and E. Green tea can protect against experimentally induced DNA damage, and slow or halt the initiation and progression of undesirable cell colonies. Studies show evidence that green tea provides immunoprotective qualities, particularly in the case of patients undergoing radiation or chemotherapy.
e. isoflavones: e.g., genistein, daidzein. Isoflavones differ from flavones by the position of the phenyl group on the benzopyrone skeleton which is α to the ketone group (3-position) and not to the ether group (2-position) and include genistein and daidzein. Isoflavones are found in soy and have an influence on bone health among postmenopausal women, together with some weak hormonal effects. Isoflavones are selectively incorporated in certain tissues like the breast and ovaries. They are able to bind to the estrogen receptors alpha (ER-alpha) and beta (ER-beta). Isoflavones acts as antioxidants to counteract damaging effects of free radicals in tissues. Isoflavones also have been found to have antiangiogenic effects (blocking formation of new blood vessels), and may block the uncontrolled cell growth associated with cancer, most likely by inhibiting the activity of substances in the body that regulate cell division and cell survival (growth factors). Studies show that groups of people who eat large amounts of soy-based products have lower incidences of breast, colon, endometrial, and prostate cancers than the general (US) population. Initial studies of soy isoflavone mixtures containing genistein, daidzein, and glycitein have found them safe for human use. Laboratory studies using animals models have shown that both soy and isoflavones can be protective against cancer when given during early life but can stimulate response to cancer-causing chemicals when given during fetal development or when circulating levels of estrogen are low.
f. anthocyanidins: e.g., cyanidin, delphinidin, malvidin. Anthocyanidins are a large water-soluble pigment group found in a large number of fruits, vegetables and flowers, and particularly in grapes and berries. These pigments give plants their brilliant colors ranging from pink through scarlet, purple and blue. Bilberry and other berries have a high concentration of anthocyanins. The anthocyanins are subdivided into the sugar-free anthocyanidine aglycons and the anthocyanin glycosides. They are considered secondary metabolites and allowed as a food additive. Scientists have identified more than 500 different anthocyanins including cyanidin, pelargonidin, delphinidin, malvidin, and paeonidin. Cyanidin, like other anthocyanidins, has antioxidant and radical-scavenging effects which, protect cells from oxidative damage and reduces the risk of heart diseases and cancer.
g. proanthocyanidins. Proanthocyanidins (also known as oligomeric proanthocyanidin OPC) form a class of flavonoid complexes found in grape seeds and grape skin, that act as antioxidants (free radical scavengers) in the human body. Thus, proanthocyanidins may help protect against the effects of internal and environmental stresses (such as cigarette smoking, pollution, and supporting normal body metabolic processes). The effects may include depressing blood fat, emolliating blood vessels, lowering blood pressure, preventing blood vessel scleroses, dropping blood viscidity and preventing thrombus formation (a type of platelet clot). Additionally, studies have shown that OPCs may prevent cardiovascular disease by counteracting the negative effects of high cholesterol on the heart and blood vessels. Proanthocyanidins are found in grapes, red wine, pine bark. Grape seed extract provides a concentrated source of polyphenols, many of which are proanthocyanidins. Also red wine is rich in proanthocyanidins. Proanthocyanidins share common properties with other polyphenols, in particular their reducing capacity and ability to chelate metal ions. However, their polymeric nature clearly makes them different. They have a high affinity for proteins and their absorption through the gut barrier is likely limited to the molecules of low polymerization degree and to the metabolites formed by the colonic microflora, as suggested by in vitro experiments. The nutritional significance of proanthocyanidins is discussed in relation to their physico-chemical properties and bioavailability.
h. procyanidins. Procyanidins (oligomeric catechins found at high concentrations in red wine, grapes and grape seeds, cocoa, cranberries, apples, and some supplements such as pycnogenol) have pronounced effects on the vascular system. Apples contain many kinds of polyphenols, and the main components are oligomeric procyanidins. Applephenon is apple polyphenol extract produced commercially from unripe apples, and has been used as food additive in order to prevent oxidation of components in foods.

### 3. Stilbenoids

Resveratrol is an exciting stilbenoid polyphenol which has potential anti-cancer and anti-aging benefits. Resveratrol is produced by plants as an antifungal chemical. It is found in the skin of grapes, in peanuts, blueberries, some pines, such as Scots pine and eastern white pine, and the roots and stalks of giant knotweed and Japanese knotweed, called hu zhang in China. It exists as two structural isomers: the cis- and trans-form. Trans-resveratrol can undergo isomerisation to the cis- form when heated or exposed to ultraviolet irradiation. Resveratrol interferes with all three stages of carcinogenesis: initiation, promotion and progression. Experiments in various cell types and isolated subcellular systems in vitro implicate a multitude of mechanisms in the pharmacological activity of resveratrol. These mechanisms include inhibition of the transcription factor NF-kB, cytochrome P450 isoenzyme CYP1A1, androgenic actions and expression and activity of cyclooxygenase (COX) enzymes. Resveratrol has been shown to induce Fas/Fas ligand mediated apoptosis, p53 and cyclins A, B1 and cyclin-dependent kinases cdk 1 and 2. Furthermore, it possesses antioxidant and anti-angiogenic properties. Due to these discoveries, resveratrol is currently being investigated extensively as a cancer chemopreventive agent. Resveratrol has recently been reported to be effective against neuronal cell dysfunction and cell death, and may be of use for diseases such as Huntington's disease and Alzheimer's disease.

### 4. Tannins

Tannins are large molecules, found in red wine, tea, and nuts. Many flavonoids in foods also occur as large molecules (tannins). These include condensed tannins (proanthocyanidins), derived tannins and hydrolysable tannins. Tannins are astringent, bitter-tasting plant polyphenols that bind and precipitate proteins. The term is applied to any large polyphenolic compound containing sufficient hydroxyls and other suitable groups (such as carboxyls) to form strong complexes with proteins and other macromolecules. Tannins have molecular weights ranging from 500 to over 20,000. Tannins are usually divided into hydrolyzable tannins and condensed tannins (proanthocyanidins). At the center of a hydrolyzable tannin molecule, there is a polyol carbohydrate (usually D-glucose). The hydroxyl groups of the carbohydrate are partially or totally esterified with phenolic groups such as gallic acid (in gallotannins) or ellagic acid (in ellagitannins). Hydrolyzable tannins are hydrolyzed by weak acids or weak bases to produce carbohydrate and phenolic acids. Condensed tannins, also known as proanthocyanidins, are polymers of 2 to 50 (or more) flavonoid units that are joined by carbon-carbon bonds, which are not susceptible to being cleaved by hydrolysis. While hydrolyzable tannins and most condensed tannins are water soluble, some very large condensed tannins are insoluble.

### 5. Monophenols: e.g., hydroxytyrosol and p-tyrosol

Hydroxytyrosol is believed to be the antioxidant with the highest free radical scavenging capacity: double that of quercetin and more than 3 times that of epicatechin. Hydroxytyrosol is the main polyphenol found in olives. The wastewaters generated during olive processing contain a high levels hydroxytyrosol, most of which can be recovered to produce hydroxytyrosol extracts. Hydroxytyrosol has the same health promoting properties as other polyphenols: prevention of atherosclerosis, promotion of intestinal and respiratory health and prevention of cancer. Hydroxytyrosol also reduces the oxidative stress caused by smoking.

### 6. Capsaicinoids

Capsaicin is the main capsaicinoid in chili peppers, followed by dihydrocapsaicin. Capsaicin was originally used in topical ointments to relieve the pain of peripheral neuropathy, for example post-herpetic neuralgia caused by Herpes zoster. Today, capsaicin creams are indicated for the temporary relief of minor aches and pains of muscles and joints associated with arthritis, simple backache, strains and sprains. With chronic exposure to capsaicin, neurons are depleted of neurotransmitters (specifically Substance P). This leads to reduction in sensation of pain and blockade of neurogenic inflammation. If capsaicin is removed, the neurons recover. The American Association for Cancer Research reports studies showing that Capsaicin is a killer of androgen-independent prostate cancer cells.

### 7. Curcumin

Curcumin is the active ingredient of the Indian curry spice turmeric. Curcumin can exist in at least two tautomeric forms, the keto and enol forms. The keto form is preferred in solid phase and the enol form in solution. Curcumin is known for its antitumor, antioxidant, anti-amyloid and anti-inflammatory properties. The anti-inflammatory action may be due to leukotriene inhibition. Curcumin acts as a free radical scavenger and antioxidant, inhibiting lipid peroxidation and oxidative DNA damage. Curcuminoids induce glutathione S-transferase and are potent inhibitors of cytochrome P450. For the last few decades, extensive work has been done to establish the biological activities and pharmacological actions of curcumin. Its anticancer effects stem from its ability to induce apoptosis in cancer cells without cytotoxic effects on healthy cells. Curcumin can interfere with the activity of the transcription factor NF-κB ( NF-kB).

In a preferred embodiment, the pharmaceutical compositions of the present invention comprises a polyphenol selected from the group consisting of the hydroxycinnamic acids, such as caffeic acid and ferulic acid; the flavones, such as luteolin and diosmin; the flavanones, such as hesperetin and naringenin; the flavonols, such as quercetin and myricetin; catechin and epicatechin; resveratrol; hydroxytyrosol; and derivatives thereof.

In a highly preferred embodiment of the present invention, the polyphenols are included in the aqueous interior of the long-circulating colloidal carriers, and preferably the long-circulating liposomes, required by the present invention. Although many polyphenols are of a lipophilic character, which would make it logical to include these in the lipophilic part of the colloidal carriers, such as in the lipophilic bilayer of the liposomes, the present inventors found that this leads to unstable compositions. In addition, the polyphenols were found to leak from systems, wherein these were incorporated in the lipophilic part.

This makes that in a highly preferred embodiment more hydrophilic polyphenols need to be used, or alternatively that the polyphenols are made water-soluble, e.g., by derivatization or complexation, for example by remote-loading

The technique of remote loading has been developed to address the potential problem associated with too hydrophobic compounds. The technique makes use of the ability of many uncharged small-molecular compounds to diffuse over the lipid bilayers. To be able to stably entrap such a compound , said compound needs to become charged after entering the liposome interior. As many drugs are weak acids or bases, this can be quite easily achieved with a so-called 'pH-shift'. This means that inside the liposome the pH is adjusted to the level at which the compound is in the charged form whereas on the outside the pH is set close to the pK of the compound so that an equilibrium towards the uncharged form is established. To further enhance stability, complexing agents or agents that form precipitates with the charged form can be added. Often with remote loading a loading efficiency of close to 100% is achieved.

A typical remote loading method encompasses the following: inside the liposome a soluble salt of the complexing/precipitating agent is encapsulated of which the counterion is able to diffuse over the lipid bilayer, for instance ammonium sulphate or calcium acetate. Sulphate is able to form crystalline complexes with several amine bases while calcium is able to precipitate a range of weak acid molecules (the uncharged counterions ammonia and acetic acid, respectively, are able to diffuse out of the liposome).

After downsizing, the unencapsulated salts are removed and the compound to-be-encapsulated is added. The pH is adjusted close (not more than 2 units away) to the pK value so that an equilibrium is created between the compound in charged form and the uncharged form. Care must be taken that the uncharged compound dose not prematurely precipitate outside the liposome. Subsequently, the temperature is raised above the lipid transition temperature allowing the uncharged compound to actually enter the lipid phase and move through the bilayer barrier into the liposome.

After entering the liposome, the uncharged compound is charged again by protonation (when the compound is a weak base) or deprotonation (when the compound is a weak acid) as a result of the intraliposomal pH. In the lipsomal interior it is in charged form able to interact with the complexing ion and form an insoluble complex or a precipitate. This will create a concentration gradient that drives the influx of new compound until all compound has entered the liposome. In reaction to this process the counterion of the complex agent will take the uncharged form and will be able to leave to the external phase, where it can be removed.

The technique of remote loading has been developed to address this potential problem. The technique makes use of the ability of many uncharged small-molecular compounds to diffuse over the lipid bilayers. To be able to stably entrap such a compound it needs to become charged after entering the liposome interior. As many drugs are weak acids or bases, this can be quite easily achieved with a so-called 'pH-shift'. This means that inside the liposome the pH is adjusted to the level at which the compound is in the charged form whereas on the outside the pH is set close to the pK of the compound so that an equilibrium towards the uncharged form is established. To further enhance stability, complexing agents or agents that form precipitates with the charged form can be added. Often with remote loading a loading efficiency of close to 100% is achieved.

Hence, the polyphenols to be encapsulated in the liposome interior can be used underivatized when sufficiently soluble in water. In most cases the water-solubility is, however, rather low, so that water soluble derivatives are preferred. Water soluble derivatives can include, but are not limited to, phosphate esters, sulphate esters, (hemi)succinate esters, glucuronides, glucosides and other sugar derivatives.

The lipid components used in forming the colloidal carriers, such as the liposomes may be selected from a variety of vesicle-forming lipids, such as phospholipids, sphingolipids and sterols. Substitution (complete or partial) of these basic components by e.g. sphingomyelines and ergosterol is possible. The liposomes in accordance with the present invention may be prepared according to general methods known in the art for the preparation of conventional liposomes and long-circulating liposomes, which includes but is not limited to lipid film hydration, solvent injection, high sheer mixing, extrusion, sonication, and homogenization.

Suitable are liposomes composed of non-charged vesicle-forming lipids, optionally including up to 20 mole percent of an amphipathic vesicle-forming lipid derivatized with a water-soluble polymer to increase long-circulating behavior and optionally including not more than 10 mole percent of negatively charged vesicle-forming lipids, the liposomes having a selected mean particle diameter in the size range between about 40 - 200 nm and containing one or more polyphenol or polyphenol derivatives entrapped in the aqueous interior.

Suitable long-circulating colloidal carriers have very favorable pharmacokinetics, a favorable tissue distribution behavior and an efficient half life. Additionally, a stable association between polyphenol and the carrier system is observed, while the loading with polyphenol is efficient. Further, a good biological availability at the site where activity is required is observed.

Passive loading of the active ingredients into the liposomes by dissolving the polyphenols or polyphenol derivatives in the aqueous interior can be sufficient in order to reach sufficient encapsulation, but other methods can also be used. To increase the efficiency of the encapsulation process liposomes can be loaded after preparation by remote loading, i.e. generating a one-way loading procedure that involves dissolving of the compound to be encapsulated in the extraliposomal aqueous environment followed by an influx of the compound into the liposome interior, which is driven by either a pH difference between the inside and the outside of the liposome bilayer or by the presence of chelators, complexing agents or agents that can cause precipitation of the compound when it enters the liposome interior. By using this method, in theory one can achieve 100% loading efficiency. This loading method can be used for polyphenols with carboxylic acid groups, such as phenolic acids, for polyphenols esterified with sulphate groups, phosphate groups and the like, but also for underivatized polyphenols that are characterized by phenol groups only. The latter can be entrapped in the liposome interior by cations such as zinc, ferric, ferrous, calcium and other non-toxic metal ions when the pH upon loading is kept sufficiently high.

In a preferred embodiment the long-circulating colloidal carrier is a liposome, a nanocapsule or a polymeric micelle; especially one that has a neutral or negative charge at physiological conditions.

Other suitable long-circulating carriers can be based on lipoproteins, and especially high density lipoproteins and low density lipoproteins, and on lipoprotein mimetics or neo-lipoproteins. It has been found that long-circulating microvesicles, and especially long-circulating liposomes, nanocapsules and polymeric micelles, are capable of efficiently delivering polyphenol drugs to a specific site, e.g., to a tumor.

The long-circulating colloidal carriers used in accordance with the present invention typically have a mean particle diameter of less than 450, and preferably less than 300 nm as determined by Dynamic light scattering using a Malvern 4700™ system equipped with a He/Ne laser, and preferably of about 40 - 200 nm.

In the most preferred embodiment, the colloidal carrier is formed by long-circulating liposomes comprising a non-charged vesicle-forming lipid, 0-20 mole percent of an amphipathic vesicle-forming lipid derivatised with polyethyleneglycol, 0-50 mole percent of a sterol, and 0-10 mol % of a negatively charged vesicle-forming lipid, which liposomes have a selected mean particle diameter in the size range between about 40 - 200 nm.

Such long-circulating liposomes are already known in the art. More particularly, these known liposome systems are described to be useful in site-specific treatment of inflammatory disorders in WO-A-02/45688. For the preparation of suitable compositions to be used in the present invention, the preparation methods described in said WO-A-02/45688 are incorporated herein by reference. In this document WO-A-02/45688, the liposome systems described in EP-A-0 662 820 are adapted to become "long-circulating".

EP-A-1 044 679 relates to liposomes having a drug included therein, which are said to have an ensured stability in blood. In addition these liposomes have an active targeting property to proteoglycan-rich areas. These areas are created because with some diseases, an over-production of proteoglycans occurs; said proteoglycans keeping cell surfaces anionic. To target liposomes to these anionic surfaces, the liposomes need to be cationic in nature. Thereto, the said liposomes require the presence of a basic compound taking positive charge within a physiological pH range.

The liposomes of the present invention do not require the active targeting property described in EP-A-1 044 679. That is, no specific homing groups are required to selectively bring the colloidal carriers containing polyphenols to the tumor sites or to sites wherein the anti-inflammatory activity is required (sites of inflammation). However, to increase the selectivity to an even higher extent, it is possible to attach or incorporate for example tumor specific antibodies or receptor ligands or food compounds at the outside surface of the carrier molecules so as to increase the interaction possibilities with the tumor cells or the cells in or around the tumor. The "targeting" of the neutral or optionally negatively charged liposomes of the present invention to tumor sites is ruled by the above-identified increased permeability in the tumor vasculature. That is, the present invention is for a major part based on passive accumulation, rather than active targeting.

Examples of inflammatory lesions for which the present invention works are inflamed joint; lesions in the central nerve system (associated with MS or other neuro-inflammatory diseases); in the liver (auto immune, hepatitis); in the intestines (Crohn's disease, collitis); in the skin and in connective tissues; in muscle tissue (polymyositis) and in or around organs showing rejections after transplantation.

The colloidal carriers, such as the liposomes useful in the present invention should not have a positive charge and should hence not comprise components that give the carriers a positive charge at physiological pH; that is at physiological pH, being a pH of between 6 and 8, the overall charge of the carrier to be used in the present invention should be neutral or negatively charged. Preferred liposomes are based on non-charged vesicle-forming lipids. Neutral or non-charged vesicle-forming lipids lead to a suitable long circulation time.

Typically, 5-10 mole% of negatively charged lipids may be present. Preferred lipids to be used to prepare the microvesicles used in the invention comprise saturated phospholipids and sphingolipids in combination with sterols, such as cholesterol and/or ergosterol and derivatives thereof. Substitution (complete or partial) of these basic components by *e.g.* sphingomyelines appeared to be possible.

To secure a suitable stability in the blood circulation system 10-50 mole% sterols should be present in the microvesicle material. Suitable liposome constituents are described in the above-identified WO-A-02/45688 and EP-A-0 662 820. More preferably, the liposomes contain at least one type of polymer lipid conjugates, such as lipids derivatized with polyalkylene glycol, preferably with polyethylene glycol (PEG). Suitable polymer-lipid-conjugates have a molecular weight of between 200 and 30,000 Dalton.

Other suitable candidates to be used in these polymer-lipid-conjugates or water-soluble polymers such as: poly ((derivatized) carbohydrate)s, water-soluble vinylpolymers (*e.g.* poly(vinylpyrrolidone), polyacrylamide and poly(acryloylmorpholine) and poly(methyl/ethyl oxazone). These polymers are coupled to the lipid through conventional anchoring molecules. Suitably, the concentration of polymer lipid conjugates is 0-20 mole%, and preferably 1-10 mole%, based upon the total molar ratio of the vesicle forming lipids. The presence of these polymer-lipid-conjugates has a favorable effect on the circulation time. However, by carefully selecting specific lipid compositions an physical specifications suitable long circulation times can be obtained without using a polymer-lipid-conjugate; for example, 50-100 nm liposomes of distearylphopshatidylcholine and cholesterol and/or sphingolipids like sphingomyelin. The liposomes may additionally contain one or more types of charged vesicle-forming lipids, e.g. phosphatidylglycerol, phosphatidylethanolamine, (di)stearylamine, phosphatidylserine, dioleoyl trimethylammonium propane, phosphatidic acids and cholesterol hemisuccinate. Typically, the concentration of charged vesicle-forming lipids is 0-15 mole%, preferably 0-10 mole% based upon the molar ratio of the vesicle forming lipids.

Polymeric micelles to be used in the present invention can be made in accordance with the method described in EP-A-1 072 617 adapted in accordance with the above-described method for the preparation of liposomes.

Where in this description reference is made to charged/uncharged/amphiphatic, and so on, this reference relates to physiological conditions.

As said, the present invention also relates to pharmaceutical compositions comprising polyphenols, encapsulated in colloidal carriers, as defined herein-above. That is, the present invention provides a medicament for or in the treatment of cancer or of anti-inflammatory or autoimmune conditions, suitable to administer polyphenols in relatively low dosages. Suitably, the medicament is a medicament for parental or local application. Application through the oral or pulmonal route are however also possible.

In accordance with the invention, effective inhibitions in tumor growth have been observed in particular embodiments with relatively low dosages of only 0.5-20 and preferably 1-10 mg/kg body weight per week

For the dosages to give an anti-inflammatory effect or for the dosages give an effect in view of autoimmune.conditions. Similar ranges are given as an example.

A composition used in accordance with the present invention may comprise one or more additional components:

Compositions to be used in accordance with the present invention may suitably contain or comprise at least one compound selected from the group consisting of cytostatic agents and cytotoxic agents, preferably at least one compound selected from the group consisting of doxorubicin and taxol.

Moreover, suitable use can be made of compositions comprising at least one component selected from the group consisting of immunomodulators and immunosuppressants. Examples of such components are methotrexate, cyclophosphamide, cyclosporin, muramyl peptides, cytokines and penicillamine.

The present invention will now be described in more detail, while referring to the following examples. In the examples, reference will be made to figure, wherein:

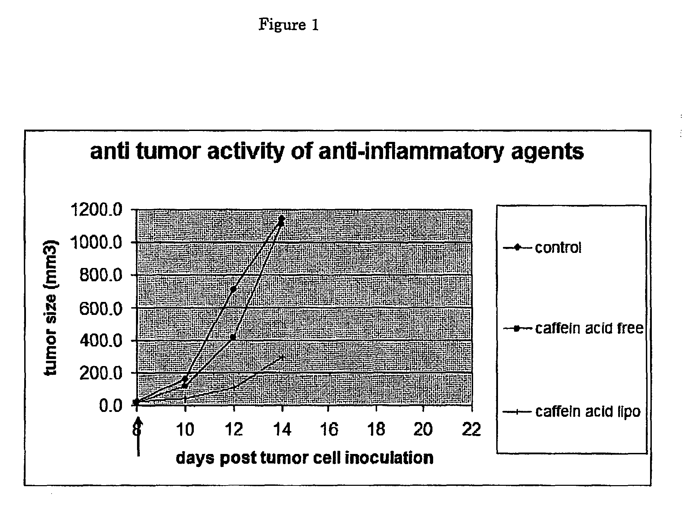

Figure 1 shows the effect of caffeine acid on tumor size.

Any percentages mentioned are percentages by weight drawn to the final composition, unless otherwise indicated.

### Example 1 - Liposome preparation

Long-circulating liposomes were prepared by dissolving dipalmitoylphosphatidylcholine (DPPC) (Lipoid GmbH, Ludwigshafen, Germany), cholesterol (Chol) (Sigma, St. Louis, USA), and poly(ethylene) glycol 2000-distearoylphosphatidylethanolamine (PEG-DSPE) (Lipoid GmbH) in a molar ratio of 1.85:1.0:0.15, respectively, in chloroform:methanol (2:1 vol:vol) in a round-bottom flask. Typically batch sizes of 1000-2000 µmol total lipid were used.

A lipid film was made under reduced pressure on a rotary evaporator and dried under a stream of nitrogen. Liposomes were formed by addition of an aqueous solution of 100 mg/ml caffeic acid (Sigma, St. Louis, USA). Liposome size was reduced by multiple extrusion steps through polycarbonate membranes (Nuclepore, Pleasanton, USA) with a final pore size of 50 nm. Unencapsulated material was removed by dialysis with repeated change of buffer against 10 mM Hepes/135 mM NaCl-buffer pH 7 at 4°C.

The mean particle size of the long-circulating liposomes was determined by dynamic light scattering to be 0.1 micrometer with a polydispersity value of 0.1. The polydispersity value varies between 0 and 1. A value of 1 indicates large variations in particle size, whereas a value of 0 indicates a complete monodisperse system. Thus, the present preparations showed limited variation in particle size.

The amount of lipid in the liposome dispersion was determined by colorimetric phosphate determination according to Rouser (Lipids 5 (1970), 494-496).

The concentration of caffeic acid in the liposomes was determined by HPLC. The liposomes contained 20-30 microgram caffeic acid/mg lipid.

### Example 2

B16 murine melanoma and C26 murine colon carcinoma cells were cultured at 37°C in a 5% CO₂-containing humidified atmosphere in DMEM medium (Gibco, Breda, The Netherlands) containing 10% (v/v) fetal calf serum supplemented with 2mM L-glutamine, 100 IU/ml penicillin, 100 microgram/ml streptomycin and 0.25 microgram/ml amphotericin B (Gibco).

Male Balb/c and C57B1/6 mice (6-8 weeks of age) were obtained from Charles River, kept in standard housing with standard rodent chow and water available ad libitum, and a 12 h light/dark cycle. Experiments were performed according to national regulations and approved by the local animal experiments ethical committee.

Mice received a single intravenous injection of an indicated dose of free caffeic acid or liposomal caffeic acid prepared in Example 1 at the time when the tumor became palpable.

At 7 days after treatment, tumor size was measured and tumor volume calculated according to the equation V = 0.52 x a² x b, wherein a is the smallest and b is the largest superficial diameter. Free caffeic acid or liposomal caffeic acid were *i*.*v*. administered at a dose of 20 mg/kg at day 1, 7 and 14 or by single injection at day 7 or day 14 after tumor cell inoculation.

As a reference, B16F10 tumors became palpable around 7 days and C26 tumors around 11 days after tumor cell inoculation.

Tumor size was measured regularly, and tumor volume was calculated as described above.

For the statistical analysis, data were analysed by one-way ANOVA with Dunnett's post test using GraphPad InStat version 3.05 for Windows, GraphPad Software (San Diego, USA). Data were logarithmically transformed to correct for significant differences between SD of groups, when appropriate according to Bartlett's test. Spearman rank correlation coefficient was calculated to identify dose-response.

To compare the effect of free caffeic acid or liposomal caffeic acid on tumor growth, B16 or C26-tumor bearing mice received a single injection of either formulation at the moment that the tumor became palpable. Between day 7 and day 14, after a second injection at day 7, liposomal caffeic acid resulted in 92% tumor growth inhibition as compared to controls (p<0.05), whereas free caffeic acid did not reduce tumor volume. On day 14, mice received a third injection. See in this light, Figure 1.

## Claims

1. A pharmaceutical composition for parenteral administration, the composition comprising long-circulating colloidal carriers comprising non-charged vesicle-forming lipids, which colloidal carriers contain one or more polyphenol or polyphenol derivatives entrapped in the carrier interior.

2. The pharmaceutical composition of claim 1, wherein the colloidal carriers have a neutral or negative charge at physiological conditions.

3. The pharmaceutical composition of claim 1 or 2, wherein the colloidal carriers are liposomes, nano-capsules or polymeric micelles.

4. The pharmaceutical composition of any one of the preceding claims, wherein the colloidal carriers have a selected mean particle diameter in the size range between about 40 - 200 nm.

5. The pharmaceutical composition of any one of the preceding claims, including up to 20 mole percent of an amphipathic vesicle-forming lipid derivatised with a water-soluble polymer.

6. The pharmaceutical composition of any one of the preceding claims, including up to 10 mol% of negatively charged vesicle-forming lipids.

7. The pharmaceutical composition of any one of the preceding claims, including up to 50 mole percent of a sterol.

8. The pharmaceutical composition of any one of the preceding claims, comprising a polyphenol selected from the group consisting of the hydroxycinnamic acids, such as caffeic acid and ferulic acid; the flavones, such as luteolin and diosmin; the flavanones, such as hesperetin and naringenin; the flavonols, such as quercetin and myricetin; catechin and epicatechin; resveratrol; hydroxytyrosol; and derivatives thereof.

9. Use of a pharmaceutical composition according to any one of the preceding claims for the treatment, and especially for the site-specific treatment of inflammatory or autoimmune disorders and cancer.

10. Use of a pharmaceutical composition according to any of the claims 1-8 for the preparation of a medicament effective in the treatment, and especially for the site-specific treatment of inflammatory or autoimmune disorders and cancer.

11. Use according to claim 11, for the site-specific treatment of inflamed tissues or regions after parenteral administration, wherein the polyphenol is used in the medicament in a water soluble form.

12. Use according to any one of claims 9-11, in the treatment of rheumatoid arthritis or of multiple sclerosis.

13. A method for loading into long-circulating colloid carriers, a polyphenol or polyphenol derivative having a deprotonatable hydroxyl group, said method comprising: (a) preparing a suspension of long-circulating colloid carriers, preferably liposomes, having a greater concentration of metal cations inside the liposomes than outside the liposomes, said metal cations being capable of forming complexes or precipitates at relatively high pH with the negatively charged polyphenol or polyphenol derivatives, (b) adding polyphenol or polyphenol derivative compounds to the said suspension, and (c) achieving uptake of the compound into the liposomes, to a final concentration of compound within the liposomes which is greater than that outside the liposomes.
